# EUROPEAN PATENT APPLICATION

(11) **EP 3 878 464 A1**
(43) Date of publication of application: **15.09.2021**
(21) Application number: 20161745.3
(22) Date of filing: 09.03.2020
(51) Int. Cl.: A61K 39/00, C07K 14/725, C07K 16/28

(54) **USE OF A CAR CELL HAVING CROSSLINKED DISULFIDE BRIDGE ON ANTIGEN RECOGNIZING MOIETY FOR TARGETING CANCER CELLS**

(71) Applicant: Miltenyi Biotec B.V. & Co. KG, 51429 Bergisch Gladbach (DE)
(72) Inventor: The designation of the inventor has not yet been filed
(74) Representative: Kisters, Michael Marcus

(57) **Abstract**

The invention is directed to the use or a method of using of a cell expressing a chimeric antigen recognition structure (CAR) bound to an adapter molecule, wherein:
i) the CAR structure comprises
a) an antigen binding domain specific for the tag of the adapter molecule,
b) a transmembrane domain
c) an intracellular domain
wherein the
ii) the adapter molecule comprises
a) a tag which is specific for the antigen binding domain of the CAR structure
b) a polypeptide comprising an antigen recognizing domain specific for an antigen expressed on the target cell, wherein the polypeptide comprises a heavy chain and a light chain connected by a sulphur atom-linker-sulphur atom bridge and wherein the linker is covalently bound to a single tag

for targeting target cells, especially human tumor cells.

## Description

### BACKGROUND

The present invention is directed to the use of CAR-cells (e.g. T cells, NK cells, B cells etc.) provided with an adapter molecule comprising an antigen recognizing domain and a site-specific reduction-resistant cross-linked disulfide-bridge and preferentially a single tag per adapter molecule for targeting cells, such as cancer cells, tumor derived (non-cancerous) cells, cells causing auto-immune diseases and soluble factors derived from tumor microenvironment or auto-immune disease.

### PRIOR ART

"Universal" CAR systems allow for addressing different antigens using the same CAR and several systems have been described, thus far.

In WO2015058018A1 the CAR binding motif expressed at the cell surface binds a desired target binding molecule in a non-specific manner, where the binding motif of the CAR is CD16 which is a low affinity Fc receptor that binds unspecifically to Immunoglobulin G antibodies. It is a disadvantage of this system that antibodies in vivo act as natural competitors to the administered antibody of interest supposed to enable specific tumor target lysis upon binding by the CAR T cells.

In WO2012082841A2 a universal, yet adaptable, anti-tag chimeric antigen receptor (AT-CAR) system is disclosed which provides T cells with the ability and specificity to recognize and kill target cells, such as tumor cells, that have been marked by tagged antibodies, e.g. FITC- or biotin labeled antibodies. Here, the disadvantage is that the labels are conjugated to the antibodies by stochastic conjugation, resulting in variable degrees of tags attached to a single antibody, which can result in non-desired activation of CAR cells
The same universal CAR system is disclosed in WO2013044225A1 facing the same disadvantages.

In WO2014100615A1 a CAR system is disclosed that makes use of CARs that target a moiety that is not produced or expressed by cells of the subject being treated. This CAR system thus allows for focused targeting of the cytotoxic lymphocytes to target cells, such as cancer cells. The targeted moiety is part of a small conjugate molecule (SCM) that also comprises a ligand of a tumor cell receptor. Because small organic molecules are typically used as the targeted moiety, clearance of the SCM from the bloodstream can be achieved within about 20 minutes. By administration of a SCM along with the CAR-expressing cytotoxic lymphocytes, the lymphocyte response can be targeted to only those cells expressing the tumor receptor, thereby reducing off-target toxicity, and the activation of lymphocytes can be more easily controlled due to the rapid clearance of the SCM. The CAR-expressing lymphocytes can also be used as a "universal" cytotoxic cell to target a wide variety of tumors without the need to prepare separate CAR constructs. As the target moieties can be FITC or biotin, this system faces the same disadvantages as WO2012082841A2 and/or WO2013044225A1.

In WO2015057852A1 switches are disclosed for regulating the activity of a chimeric antigen receptor effector cells (CAR-ECs). These switches generally comprise a chimeric antigen receptor-interacting domain (CAR-ID) and a target interacting domain (TID). The CAR-ID may be e.g. FITC or biotin.

This system features site-specific labeling of target cell binding moieties but relies on co-translational incorporation of non-natural amino acids. Again, this system faces the same disadvantages as WO2012082841A2 and/or WO2013044225A1.

In WO2015057834A1 a chimeric antigen receptor-effector cell switch is disclosed comprising: a) a peptidic antigen that binds a chimeric antigen receptor on an effector cell; and b) a targeting moiety that binds a cell surface molecule on a target cell. The peptidic antigen is recombinantly expressed and based on or derived from a naturally occurring peptide or from a non-naturally occurring peptide. The disadvantage of this system is that there is a high risk of autoimmunity by tags derived from a human nuclear protein.

In WO2016030414A1 a universal chimeric antigen receptor is disclosed, wherein the receptor comprises three domains, wherein the first domain is a tag-binding domain, the second domain is a linking peptide chain including an extracellular hinge and a transmembrane domain and the third domain is a signal transduction domain, wherein the tag-binding domain binds to a tag derived from any human nuclear protein. In particular suitable tags are peptide sequences from nuclear antigens, which cannot be accessed and bound by the corresponding tag-binding domain in the context of the native protein under physiological conditions. A disadvantage of this system is that the tag can only be added in a co-translational manner, thereby excluding the use of established monoclonal antibodies as tagged antigen binding molecules without prior redesign of the gene encoding said antigen binding molecule.

Therefore, there is a need in the art for an improved or alternative universal (adaptable) CAR system. Which allows the combination of post- translational, site-specific labeling technology which allows attachment of a tag or affinity unit at a preferred site of the proteinogenic target cell binding moieties for optimal functionality of the system.

From a different technology field, crosslinking a disulfide bridge of an antibody is known. For example the chemistry of recognition of two neighboring reduced thiols previously forming a disulfide bridge was first described in a scientific publication in 1979 at Sumita Mitra et al. "Reagents for Cross-Linking of Proteins", JACS 101211 (1979), Liberatore F. et al. Bioconjugate Chem, 1, (1990), and Rosario et al. Bioconjugate Chem, 1990, 1, 51-59; where the crosslinker was referred to as: equilibrium transfer alkylation crosslinker (ETAC). It is described that a full antibody is reacted with a cross linker for the purpose of labeling the antibody with a fluorescent dye. Another scientific publication describes a similar use on a Fab (Wilbur et al., Bioconjugate Chem 1994, 5, 220-235).

US 2016/0000933 A1 discloses the crosslinking of antibodies at their disulfide bridge for the preparation of antibody conjugates.

Further publications pertinent to crosslinking of antibodies at their disulfide bridge are for example WO 2016/168769 A1; HANIEH KHALILI ET AL: "Comparative Binding of Disulfide-bridged PEG-Fabs". BIOCONJUGATE CHEMISTRY, vol. 23, no. 11, 21 September 2012 (2012-09-21), pages 2262-2277; Greg T. Hermanson: "Antibody Modification and Conjugation" In: "Bioconjugate Techniques", 1 January 2013 (2013-01-01), Academic Press, ISBN: 978-0-12-382239-0 pages 867-920; WO 2016/154621 A1

CAR-T cells are genetically engineered cells that express a chimeric antigen receptor (CAR) moiety on their surface which is used to recognize and target unwanted cells. Upon binding, the CAR triggers an activation signal on the CAR-T cells to kill the target cells.

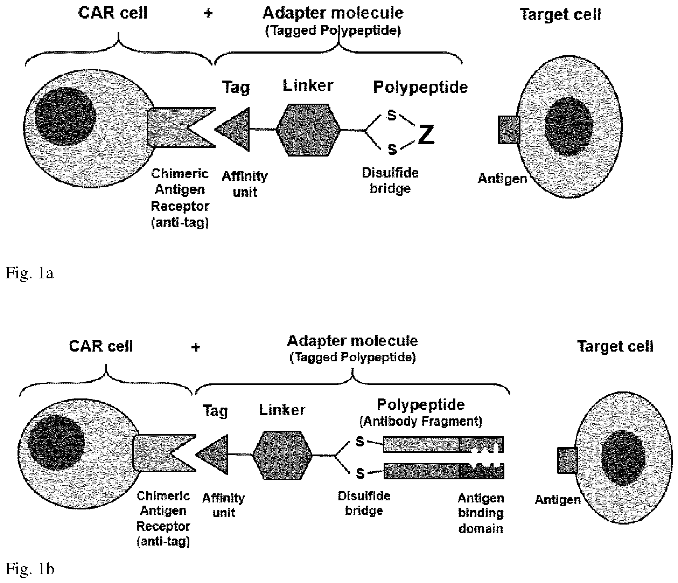

In order to mediate the recognition and binding between CAR-T cells and the target cells, an adapter molecule is required. An universal CAR T cell adapter molecule is composed of an antibody or antibody fragment (e.g. Fab) specific to recognize an antigen on the target cells plus a secondary moiety (commonly referred to as tag or affinity unit) which is recognized only by the CAR-T cells. This way, the adapter molecule serves as a recognition bridge between the target cells and the CAR-T cells (see Fig. 1a). By using Ab and Fabs different cell populations can be targeted (Fig. 1b). And the CAR T cells are only functional against target cells in the presence of the adapter molecule.

Currently, universal affinity tags that are recognized by the CAR-T cells are Vitamins (e.g. Biotin), which are naturally occurring ones and non-biologically orthogonal, e.g. FITC .

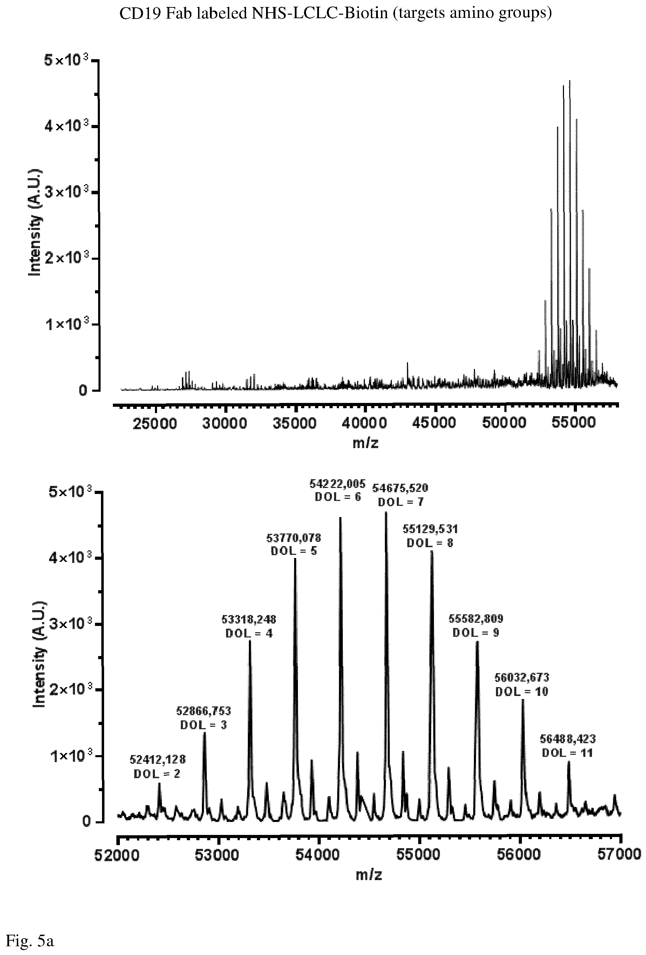

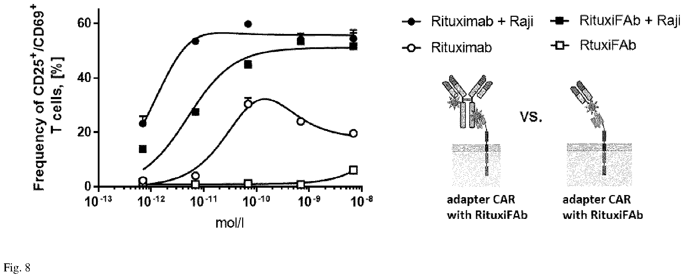

The problem of anti-adapter-CAR-T-cells and tagged antibodies comes in the formulation and design of the CAR-T cell adapter molecule. Current preparations are based on classical non-site-specific labeling strategies, i.e. stochastic biotinylation of antibodies (Ab) or Fabs by targeting amino groups on lysines with NHS-ester biotin reagents. This produces conjugates with a stochastic site-labeling of biotin on the Ab or Fab, with a distribution on the number of biotins per Ab and Fabs ranging between 1 to 20 biotins per Ab or Fab (see Fig. 5a). The result is a group of non-well-defined adapter molecules which translates in a non-optimal performance and which can activate CAR cells even in the absence of target cells (see Fig. 8). Even of greater relevance, is that by crosslinking the heavy and light chains of antibodies and antibody fragments and re-bridging the interchain disulfide bonds, the resulting bond becomes resistant to reduction and reducing environments, such as the ones that can be found in tumor microenvironments (TME), thus stabilizing the adapter molecule.

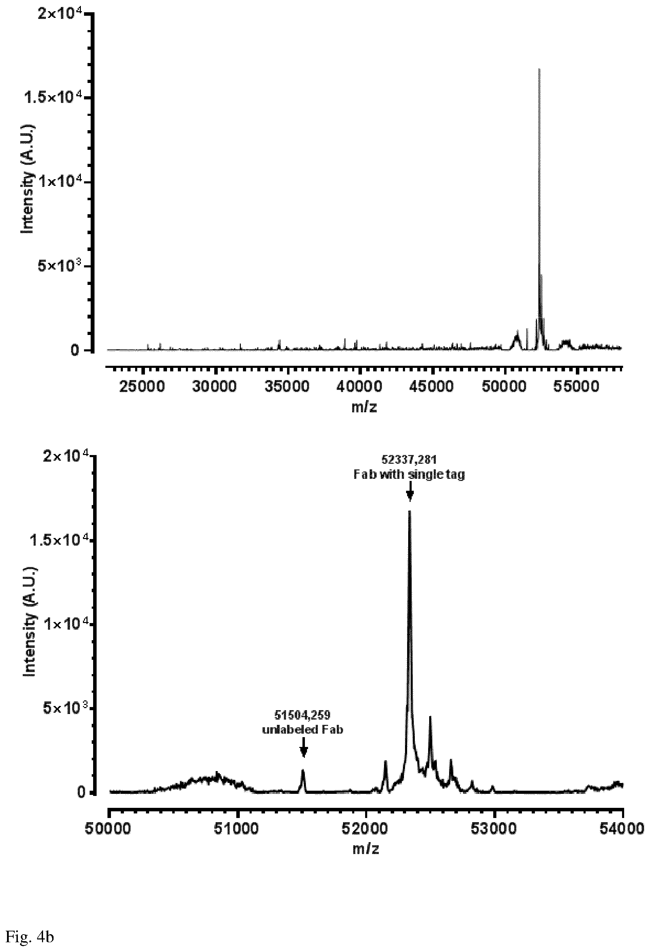

It was therefore an object of the invention to provide a chemically well-defined adapter molecule with an univariant number of tag / affinity units (e.g. Biotin) per Ab and/or Fab on site-specific positions (example shown on Fig. 4b), where both chains are crosslinked via a covalent bridge resistant to thiol reduction, which allows for optimal sensitivity and functionality.

### SUMMARY OF THE INVENTION

The invention is based on using a site-directed cross - linker that targets disulfide bonds on Ab and/or Fabs. The benefit relies on: i) specific location of disulfide bridge, which is known in the sequence and structure of Ab and Fabs at fixed positions; ii) the crosslinker covalently attaches to both reduced thiols of the disulfide bridge, thus preserving the native 3D structure and function of the Ab and Fab leaving no free thiols behind; iii) each crosslinker carries only one tag or affinity unit moiety (e.g. Biotin or Thiamine), so for each disulfide bridge will be modified by linker carrying one biotin providing a known and predictable stoichiometry (i.e. fabs only contain one disulfide bridge at the C-terminal); iv) the crosslink reaction is quantitative, meaning above 95% of Ab and Fab is labeled; v) degree of labeling can be stoichiometric controlled (i.e. for Ab); vi) the crosslink conjugate is insensitive to thiol reduction and reducing environments.

Accordingly, object of the invention is the use of a cell expressing a chimeric antigen recognition structure (CAR) non-covalently bound to an adapter molecule, wherein:
i) the CAR structure comprises
   a) an antigen binding domain specific for the tag of the adapter molecule,
   b) a transmembrane domain
   c) an intracellular domain
   wherein the
ii) the adapter molecule comprises
   a) a tag which is specific for the antigen binding domain of the CAR structure
   b) a polypeptide comprising an antigen recognizing domain specific for an antigen expressed on the target cell, wherein the polypeptide comprises a heavy chain and a light chain connected by a sulphur atom-linker-sulphur atom bridge and wherein the linker is covalently bound to a single tag
for targeting target cells.

The use of CAR cells as disclosed later on has the following advantages
- The interchain disulfide bonds stabilizes the quaternary structure of antibodies and antibody fragments.
- Prevention of reduction of disulfide bonds of antibodies or antibody fragments to sulfhydryl groups, and thereby losing their contribution to stabilization of antibodies or antibody fragments quaternary structure.
- Prevention of reduction avoids dissociation of antibodies or antibody fragments into individual chains. This process, may only be partially reversible, depending on the nature of the antibody fragment and contribution to other stabilizing interactions.

Therefore, replacement of the interchain disulfide bond by a reduction insensitive disulfide linker can render antibody or antibody fragment-based adapter molecules to be less reduction sensitive. This might be of particular advantage for CAR adapter molecules that are stored, resuspended, and injected into patients, i.e. experience different environments, and advantageous against other adapters in the context of tumor microenvironments are known to exhibit different reduction potential environments and could negatively impact the adapter stability and thus the universal CAR cell therapy efficacy.

### DEFINITIONS

The terms "adapter", "adapter molecule", "affinity unit" or "tagged polypeptide" are here used indistinctively and refer to units comprising a tag (i.e. affinity unit, hapten) which is specific recognized by the antigen binding domain of the CAR structure by forming a non-covalent bond.
The terms "a tag which is specific for the antigen binding domain of the CAR structure" and " antigen binding domain specific for the tag of the adapter molecule" refer to a so called affinity system wherein a non-covalent bound is established between the CAR cell and adapter molecule reaction partners. Examples for such systems are disclosed in the following.
The term "linker" refers to any residue which can be obtained by the reaction with a reduced disulfide bond between the heavy chain and a light chain of a polypeptide, including but not limited to antibodies (Ab) and antibody fragments (Fab). Examples for such systems are disclosed in the following.
The terms "having specificity for", "specifically binds" or "specific for" with respect to an antigen-binding domain of an adapter molecule or of a CAR refer to an antigen-binding domain which recognizes and binds to a specific antigen, but does not substantially recognize or bind other molecules in a sample. An antigen-binding domain that binds specifically to an antigen from one species may bind also to that antigen from another species. This cross-species reactivity is not contrary to the definition of that antigen-binding domain is specific. An antigen-binding domain that specifically binds to an antigen may bind also to different allelic forms of the antigen (allelic variants, splice variants, isoforms etc.). This cross reactivity is not contrary to the definition of that antigen-binding domain is specific.

### BRIEF DESCRIPTION OF THE DRAWINGS

Fig. 1a : general concept of the CAR cell and adapter molecule of the invention
Fig. 1b: a specific example of the general concept where the polypeptide in the adapter molecule construct is an antibody fragment.
Fig. 1c: a specific example of the general concept where the polypeptide is an antibody fragment and the linker is from the family of equilibrium transfer alkylation reagents in the tagged polypeptide (adapter molecule) construct
Fig. 2a: a specific example of the general concept where the polypeptide is an antibody fragment, the linker is from the family of equilibrium transfer alkylation reagents, and the tag (affinity unit) is Biotin in the tagged polypeptide (adapter molecule) construct
Fig. 2b: a specific example of the general concept where the polypeptide is an antibody fragment, the linker is from the family of equilibrium transfer alkylation reagents, and the tag (affinity unit) is Thiamin in the tagged polypeptide (adapter molecule) construct
Fig. 3a: a specific example of the general concept where the polypeptide is an antibody fragment and the linker is from the family of dihalogenated maleimide reagents in the tagged polypeptide (adapter molecule) construct
Fig. 3b: a specific example of the general concept where the polypeptide is an antibody fragment and the linker is from the family of pyridazedione reagents in the tagged polypeptide (adapter molecule) construct
Fig. 4 to 10: Results of the experiments / examples

### DETAILED DESCRIPTION

The general concept of the invention is shown in Fig.1, showing a cell bearing a chimeric antigen receptor ("CAR") with the sequence of units signaling domain (not shown), transmembrane domain (not shown), chimeric antigen receptor unit (all depicted as X), and a tagged polypeptide comprising a: tag (affinity unit), linker unit and polypeptide with an antigen recognizing domain.

As already pointed, there are numerous patent publications disclosing CAR cells CAR-T cell adapter molecule technology. Insofar, a person skilled in the art is aware how to obtain a cell e.g. a chimeric antigen receptor ("CAR") cell with the sequence of units signaling domain (not shown), transmembrane domain (not shown), chimeric antigen receptor unit (all depicted as X). Preferable, the cell is a T-Cell, B-cell, NK cell, dendritic cell, tumor infiltrating lymphocyte (TIL) or macrophage.

The CAR structure comprises, an antigen binding domain specific for the tag of the adapter molecule, a transmembrane domain, and an intracellular domain. Generally, an "antigen binding domain" refers to the region of the CAR that specifically binds to an antigen, e.g. to a cell bound or soluble antigen as disclosed herein. Generally, the targeting regions on the CAR are extracellular. The antigen binding domain may comprise, for example, full length heavy chain, Fab fragments, single chain Fv (scFv) fragments, divalent single chain antibodies or diabodies. Any molecule that binds specifically to a given antigen such as affibodies or ligand binding domains from naturally occurring receptors may be used as an antigen binding domain. Often the antigen binding domain is a scFv. Normally, in a scFv the variable regions of an immunoglobulin heavy chain and light chain are fused by a flexible linker to form a scFv. Such a linker may be for example the "(G4/S)3-linker". In some instances, it is beneficial for the antigen binding domain to be derived from the same species in which the CAR will be used in. For example, when it is planned to use it therapeutically in humans, it may be beneficial for the antigen binding domain of the CAR to comprise a human or humanized antibody or antigen binding fragment thereof. Human or humanized antibodies or antigen binding fragments thereof can be made by a variety of methods well known in the art. "Hinge" as used herein refers to the hydrophilic region which is between the antigen binding domain and the transmembrane domain. The CARs of the invention may comprise an extracellular hinge domain but it is also possible to leave out such a hinge. The hinge may include e.g. Fc fragments of antibodies or fragments thereof, hinge regions of antibodies or fragments thereof, CH2 or CH3 regions of antibodies, accessory proteins, artificial spacer sequences or combinations thereof. A prominent example of a hinge is the CD8alpha hinge. The transmembrane domain of the CAR may be derived from any desired natural or synthetic source for such domain. When the source is natural the domain may be derived from any membrane-bound or transmembrane protein. The transmembrane domain may be derived for example from CD8alpha or CD28. When the key signaling and antigen recognition modules (domains) are on two (or even more) polypeptides then the CAR may have two (or more) transmembrane domains. The cytoplasmic signaling domain (the intracellular signaling domain or the activating endodomain) of the CAR is responsible for activation of at least one of the normal effector functions of the immune cell in which the CAR is expressed, if the respective CAR is an activating CAR (normally, a CAR as described herein refers to an activating CAR). "Effector function" means a specialized function of a cell, e.g. in a T cell an effector function may be cytolytic activity or helper activity including the secretion of cytokines. The intracellular signaling domain refers to the part of a protein which transduces the effector function signal and directs the cell expressing the CAR to perform a specialized function. The intracellular signaling domain may include any complete, mutated or truncated part of the intracellular signaling domain of a given protein sufficient to transduce a signal which initiates or blocks immune cell effector functions. Prominent examples of intracellular signaling domains for use in the CARs include the cytoplasmic signaling sequences of the T cell receptor (TCR) and co-receptors that initiate signal transduction following antigen receptor engagement. Generally, T cell activation can be mediated by two distinct classes of cytoplasmic signaling sequences, firstly those that initiate antigen-dependent primary activation through the TCR (primary cytoplasmic signaling sequences, primary cytoplasmic signaling domain) and secondly those that act in an antigen-independent manner to provide a secondary or co- stimulatory signal (secondary cytoplasmic signaling sequences, co-stimulatory signaling domain). Therefore, an intracellular signaling domain of a CAR may comprise one or more primary cytoplasmic signaling domains and/or one or more secondary cytoplasmic signaling domains. Primary cytoplasmic signaling domains that act in a stimulatory manner may contain ITAMs (immunoreceptor tyrosine-based activation motifs). Examples of ITAM containing primary cytoplasmic signaling domains often used in CARs are that those derived from TCRab (CD3z), FcRgamma, FcRbeta, CD3gamma, CD3delta, CD3epsilon, CD5, CD22, CD79a, CD79b, and CD66d. Most prominent is sequence derived from CD3z (CD3zeta). The cytoplasmic domain of the CAR may be designed to comprise the CD3z signaling domain by itself or combined with any other desired cytoplasmic domain(s). The cytoplasmic domain of the CAR can comprise a CD3z chain portion and a co-stimulatory signaling region (domain). The co-stimulatory signaling region refers to a part of the CAR comprising the intracellular domain of a co-stimulatory molecule. A co-stimulatory molecule is a cell surface molecule other than an antigen receptor or their ligands that is required for an efficient response of lymphocytes to an antigen. Examples for a co-stimulatory molecule are CD27, CD28, 4-1BB (CD137), OX40, CD30, CD40, PD-1, ICOS, lymphocyte function-associated antigen- 1 (LFA-1), CD2, CD7, LIGHT, NKG2C, B7-H3. The cytoplasmic signaling sequences within the cytoplasmic signaling part of the CAR may be linked to each other with or without a linker in a random or specified order. As an example, the cytoplasmic domain may comprise the signaling domain of CD3z and the signaling domain of CD28. In another example the cytoplasmic domain may comprise the signaling domain of CD3z and the signaling domain of CD137. In a further example, the cytoplasmic domain may comprise the signaling domain of CD3z, the signaling domain of CD28, and the signaling domain of CD137.

Preferable, the cell expressing a chimeric antigen recognition structure (CAR) is a T-Cell, B-cell, NK cell, dendritic cell, tumor infiltrating lymphocyte or macrophage .

The tag may be e.g. affinity unit or a hapten which may be bound by the CAR, comprising an antigen binding domain specific for the tag. Haptens such as e.g. FITC, biotin, thiamin, PE, streptavidin or dextran are small molecules that elicit an immune response only when attached to a large carrier such as a protein; the carrier may be one that also does not elicit an immune response by itself. But the tag may also be a peptide sequence. The peptide may be selected from the group consisting of c-Myc-tag, Strep-Tag, Flag-Tag, and Polyhistidine-tag. The tag may also be streptavidin. The tag portion of the tagged polypeptide is only constrained by being a molecule that can be recognized and specifically bound by the antigen binding domain specific for the tag of the CAR. For example, when the tag is FITC (Fluorescein isothiocyanate), the tag-binding domain may constitute an anti-FITC scFv. Alternatively, when the tag is biotin or PE (phycoerythrin), the tag-binding domain may constitute an anti-biotin scFv or an anti-PE scFv.

The tags/affinity units used in the present invention are for example selected from the group consisting of amino acids, peptides, proteins, creatinine, biotin, biocytin, thiamin, fluorochromes, lipids, hormones, vitamins, carbohydrates.

Since the affinity unit shall bind specifically to the chimeric antigen receptor unit, a person skilled in the art will have no difficulty to choose the appropriate chimeric antigen receptor unit for the adapter. By way of example, if biotin is selected as affinity unit, the scFv of an anti-biotin antibody or anti-biotin Fab should be selected as the chimeric antigen receptor unit.

### Use of the invention

The term "targeting cells" refers to recognizing, detecting, killing or at least manipulating target cells. Preferable, such target cells origin from a solid tumor and/or from tumor microenvironment (TME) of a solid tumor. Such tumor cells may be human tumor cells. The CAR cell and adapter molecule may also target non-cancerous cells e.g. T cells or B cells which belong to a solid tumor or a specific tumor microenvironment. Further, CAR cell and adapter molecules may also be directed to soluble factors , e.g. proteins derived from the tumor microenvironment.

Furthermore, target cells may originate from auto-immune disease and/or the adapter molecules bind and are specific for soluble antigens.

The advantage of such cells is that due to the adapter technology, "standard CAR" cells can be easily converted into specific CAR cells, equipped with a specific antigen recognizing moiety usually found on antibodies or antibody fragments to attack target cells. Accordingly, cells of the invention may be used in cellular therapy wherein the recipient may be the same subject from which the cells were obtained (autologous cell therapy) or from another subject of the same species (allogeneic cell therapy). In these therapies, the cells together with the appropriate adapter molecule of the invention are administered to a recipient and are then able to kill or at least stop growth of target cells expressing the antigen which is recognized by the cells according to the invention.

The target cell or target moiety to be recognized with the aid of the invention can be on any biological specimen, like tissues slices, cell aggregates, suspension cells, or adherent cells. The cells may be living or dead. Preferable, target moieties are antigens expressed extracellular on biological specimen like whole animals, organs, tissues slices, cell aggregates, or single cells of invertebrates, (e.g., Caenorhabditis elegans, Drosophila melanogaster), vertebrates (e.g., Danio rerio, Xenopus laevis) and mammalians (e.g., Mus musculus, Homo sapiens). More preferred target moieties are found on cancer cells in a human subject.

Such cancer cells (i.e. target cells) may be derived from leukemia, acute myeloid leukemia, Non-Hodgkin lymphoma, Hodgkin lymphoma, multiple myeloma, adrenal cancer, anal cancer, bile duct cancer, bladder cancer, bone cancer, brain/CNS tumors in children or adults, breast cancer, cervical cancer, colon/rectum cancer, endometrial cancer, esophagus cancer, ewing family of tumors, eye cancer, gallbladder cancer, gastrointestinal carcinoid tumors, gastrointestinal stromal tumor (GIST), gestation trophoblastic disease, hodgkin disease, kaposi sarcoma, kidney cancer, laryngeal and hypopharyngeal cancer, leukemia, acute lymphocytic leuckemia, acute myeloid leukemia, chronic lymphocytic leukemia, chronic myeloid leukemia, chronic myelomonocytic leukemia, liver cancer, lung cancer, non-small cell lung cancer, small cell lung cancer, lung carcinoid tumor, lymphoma, malignant mesothelioma, multiple myeloma, myelodysplastic syndrome, nasal cavity and paranasal sinum cancer, nasopharyngeal cancer, neuroblastoma, non-hodgkin lymphoma, oral cavity or oropharyngeal cancer, osteosarcoa, ovarian cancer, pancreatic cancer, penile cancer, pituitary tumors, prostate cancer, retinoblastoma, rhabdomyosarcoma, salivary gland cancer, skin cancer, sarcoma, basal skin cancer, squamous cell skin cancer, melanoma, merkel cell skin cancer, small intestine cancer, stomach cancer, testicular cancer, thymus cancer, thyroid cancer, uterine sarcoma, vaginal cancer, vulvar cancer, Waldenstrom macroglobulinemia, or wilms tumor and/or the respective tumor microenvironment (TME).

Further target cells may be related to autoimmune diseases such as Behcet's disease, Juvenile idiopathic arthritis, Type 1 diabetes, Rheumatoid arthritis, Wegener Granulomatosis, Systemic lupus erythematosus, Systemic sclerosis , Crohn's disease, Graves' disease, Hashimoto thyreoiditis, Goodpasture syndrome, pernicieuse anemia, Primary biliary cholangitis, Myasthenia gravis, Dermato polymyositis, Vasculitis, Mixed connective tissue disease, Scleroderma, Multiple sclerosis, Psoriasis, Ulcerative colitis or Uvetis.

Further, the TME in solid tumors, tissue hypoxia as consequence of imbalanced angiogenesis is prevalent and is associated with changes in metabolic pathways, including a higher dependence on glycolysis resulting in tissue acidosis. Both hypoxia and acidosis affect the tissue redox status. Overall it might be expected that stabilization of a disulfide bond in antibody fragments-based adapters might render those molecules less susceptible to the reducing potential of TME.

Therefore disulfide-crosslinking might represent a beneficial feature for therapeutic application of adapter molecules and may be of special relevance for targeting TME of solid tumors.

In a first variant of the invention the cell expressing a chimeric antigen recognition structure (CAR) is a T cell; the antigen binding domain specific for the tag is anti-biotin; the tag is biotin; and the linker is derived from the family of equilibrium transfer alkylation reagents (ETAC), and the polypeptide is an antibody fragment (Fab molecule/ fragmented antibody).

In a second variant of the invention the cell expressing a CAR is a T cell; the antigen binding domain specific for the tag is anti-thiamin; the tag is thiamin; and the linker is derived from the family of equilibrium transfer alkylation reagents (ETAC), and the polypeptide is an antibody fragment (Fab molecule/fragmented antibody)

### Antigen recognizing domain

The term "antigen recognizing domain " refers to any kind of antibody, fragmented antibody or fragmented antibody derivatives, directed against a target moiety expressed on a biological specimen of interest. The term relates to fully intact antibodies, fragmented antibody or fragmented antibody derivatives, e.g., Fab, Fab', F(ab')2, sdAb, scFv, di-scFv, nanobodies. Such fragmented antibody derivatives may be synthesized by recombinant procedures including covalent and non-covalent conjugates containing these kind of molecules. Further examples of antigen recognizing moieties are peptide/MHC-complexes targeting TCR molecules, cell adhesion receptor molecules, receptors for costimulatory molecules, artificial engineered binding molecules, e.g., peptides or aptamers which target, e.g., cell surface molecules.

Preferable, the term "Antigen recognizing domain " refers to a moiety directed against antigen expressed by the biological specimens (target cells) extracellular, like IL2, FoxP3, CD154, CD33 (Siglec-3), CD123 (IL3RA), CD135 (FLT-3), CD44 (HCAM), CD44V6, CD47, CD184 (CXCR4), CLEC12A (CLL1), LeY, FRβ, MICA/B, CD305 (LAIR-1), CD366 (TIM-3), CD96 (TACTILE), CD133, CD56, CD29 (ITGB1), CD44 (HCAM), CD47 (IAP), CD66 (CEA), CD112 (Nectin2), CD117 (c-Kit), CD133, CD146 (MCAM), CD155 (PVR), CD171 (L1CAM), CD221 (IGF1), CD227 (MUC1), CD243 (MRD1), CD246 (ALK), CD271 (LNGFR), CD19, CD20, GD2, EGFR, and CD33. These antigen may also be soluble antigens specifically associated with the TME e.g. arginase, carcinoembryonic antigen, CCL11, CCL18, CCL2, CCL5, CD282, Circulating Tumor Nucleic Acids, CXCL10, FAP, GM-CSF, IFN-y,IL-4, IL-6, IL-7 IL-8, IL-10, IL-11, IL-12, 11-13, IL-14, IL-15, IL-17, IL-23, IL-33, IL-1beta, IL-1Ra, INF, LAP, M-CSF, MMP12, MMP13, MMP7, NY-ESO-1 antibody, prostate-specific antigen, sCD106, sCD137, sCD152, sCD223, sCD25, sCD27, sCD253, sCD270, sCD273, sCD274, sCD279, sCD28, sCD30, sCD366, sCD40, sCD54, sCD80, sCD86, sGITR, TGFβ-1, TGFβ-2, TGFβ-3, TIMP1 or TNF-α, VEGF. The "s" in sCDx regularly stands for a soluble form of the respective CDx molecule.

The term "soluble antigen" as used herein refers to an antigen that is not immobilized on surfaces such as beads or cell membranes, i.e. it is soluble during the process of binding to a adapter CAR via a tagged polypeptide as disclosed herein and of subsequently activating the immune cell that expresses said CAR. This definition does not exclude the possibility that the soluble antigen may be immobilized temporary, e.g.by temporary binding to surface of a cell membrane such as a chemokine can do. The soluble antigen is dissolved in a liquid, e.g. in an interstitial fluid of a subject.

### Linker unit

Suitable linker unit comprise at least one reactive group selected from the group consisting of the family of equilibrium transfer alkylation reagents (ETAC) with at least one bis-sulfone leaving group, of dihalogenated (e.g. dibromo, dichloro) maleimides and of pyridazediones.

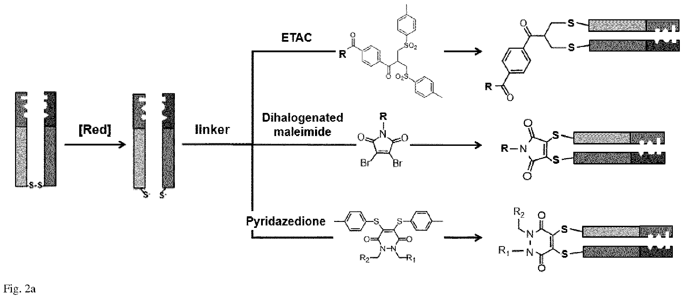

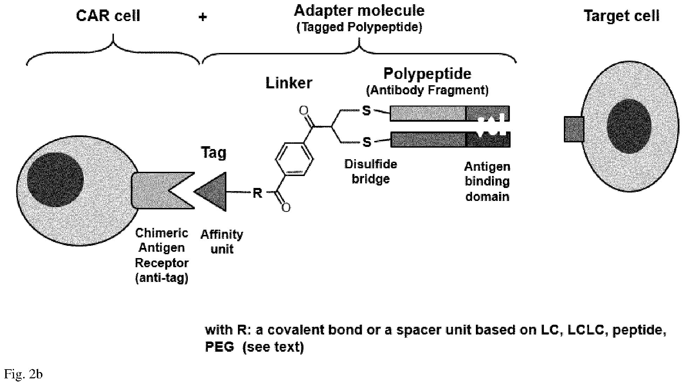

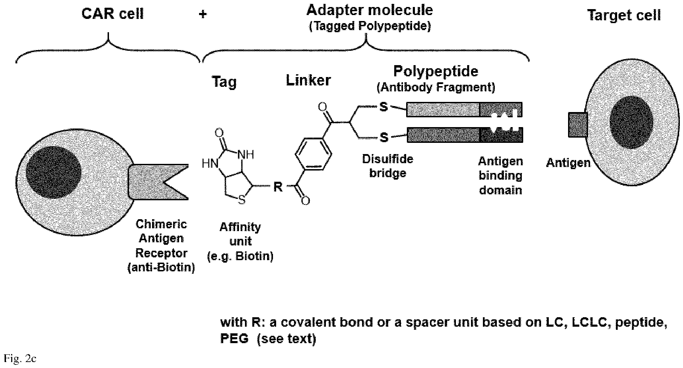

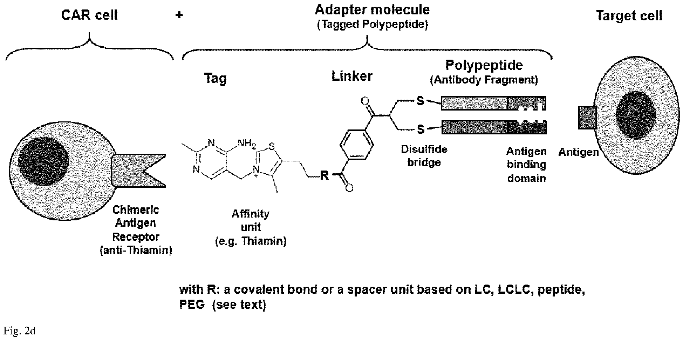

The chemistry of reacting the cross linker unit with the disulfide bond of the antigen recognizing domain is shown in Fig. 2.

### Adapter molecule

The term "adapter molecule" refers to a group or molecule comprising affinity unit, linker unit and antigen recognizing domain. It is preferred to synthesize the adapter molecule independent from the cell with an antigen recognizing structure comprising the sequence: signalling domain, transmembrane domain, chimeric antigen receptor unit.

Preferable, the adapter molecule was generated by reducing the disulfide bond between the heavy chain and a light chain of a polypeptide and subsequent reaction with the linker wherein the linker is a molecule consisting of the family of equilibrium transfer alkylation reagents (ETAC), of the family of dihalogenated maleimides reagents, of the family of pyridazedione reagents.

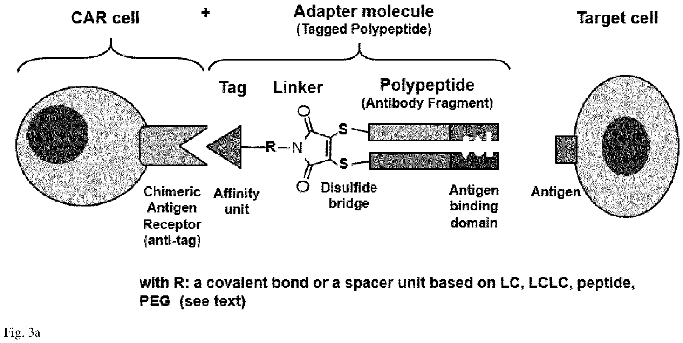

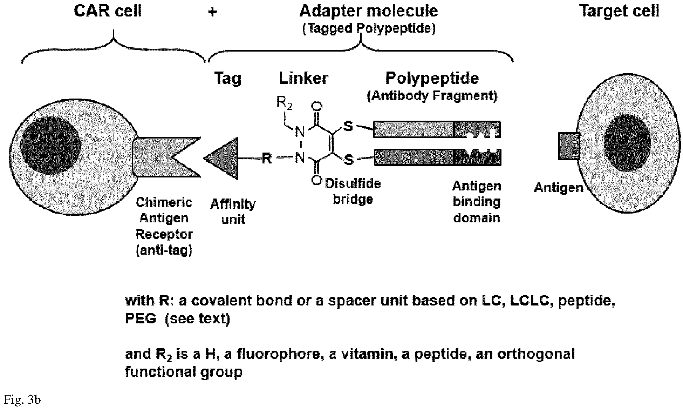

For example, such adapter molecule provided with the sequence affinity unit/ linker unit/antigen recognizing domain unit comprises the reaction of one of the compounds shown in formula I to III with a reduced antibody or antibody fragment (aka polypeptide with antigen recognizing domain) in a reaction scheme described in Fig. 2a and examples of adapter molecules on Fig 2b to 3b. 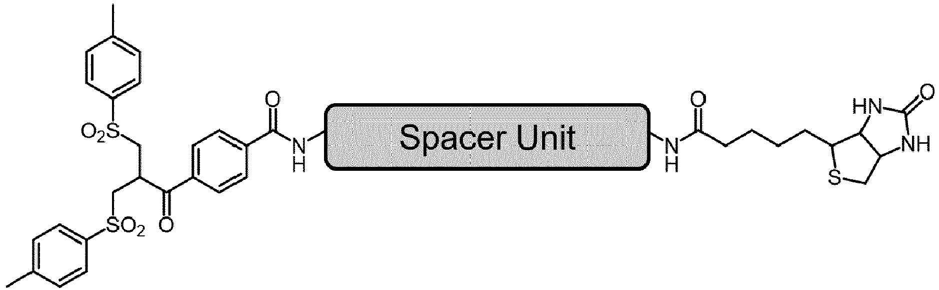
(I) with "spacer unit" = a covalent bond, PEOn (n=1-50), PEGₙ (n=1-50), peptide 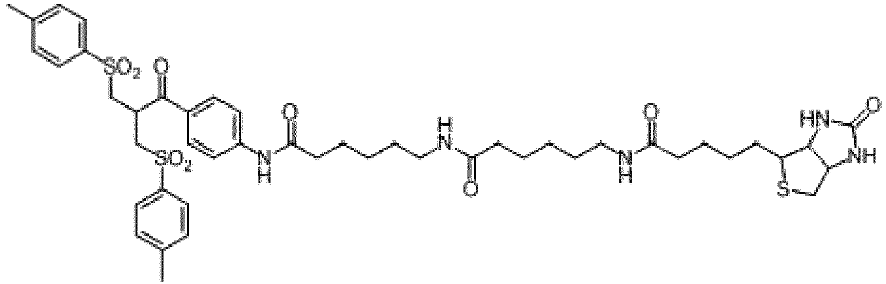
(II) with "spacer unit" = LCLC 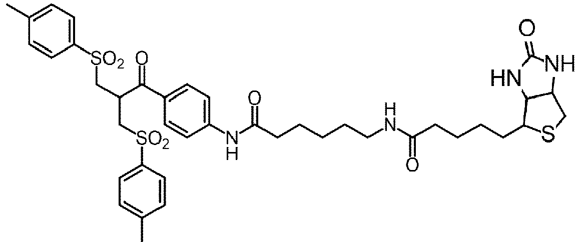
(III) with "spacer unit" = LC

In a variant of the invention, the adapter molecule is synthesize step by step (or unit by unit) starting with the affinity unit bound to the chimeric antigen receptor unit.

### Method used in the invention

The method of the invention is directed to provide a cell with an antigen recognizing structure comprising the sequence: signalling domain, transmembrane domain, chimeric antigen receptor unit, tag (affinity) unit, linker unit and antigen recognizing domain characterized in that a cell provided with an sequence comprising signalling domain, transmembrane domain, chimeric antigen receptor unit is bound to an adapter molecule comprising tag (affinity) unit, linker unit and antigen recognizing domain wherein the chimeric antigen receptor bind specifically to the tag (affinity) unit.

All features, embodiments and variants described for the cell can be employed within the method.

Another object of the invention is a method to manufacture an adapter molecule comprising a tag (affinity) unit, a linker unit (with a specific spacer unit) and one antigen recognizing domain characterized in that the antigen recognizing domain comprises a disulfide bond which is bound to the linker unit and wherein affinity unit is bound to the spacer of the linker unit.

Again, all features, embodiments and variants described for the cell can be employed within this method.

### Examples

### Example #1 of site-specific labelling and mono-functionalization of Fab andintact mass determination.

CD19 Fab was used at a concentration of 1g/L in PEB buffer (PBS + EDTA buffer). DTT was added as a reducing agent for 1 hour. Then removed by buffer exchange and the ETAC crosslinker molecule added in a 1,8 fold excess and allow to react for 2-3 hours. After that the sample was buffer exchanged. To prepare the samples for LC-MS analysis, concentration was adjusted to 300µg/ml by absorbance for both CD19Fab unmodified (control) and CD19Fab-monoBiotin. The LCMS results show that in both cases there is only one significant peak corresponding to the full Fab, which in case of the control has a mass of 51506,394 Dalton, and in the case of the modified Fab it shifts to 52337,281 Dalton, with the mass difference corresponding to the added mass by a single crosslinker molecule per Fab unit.

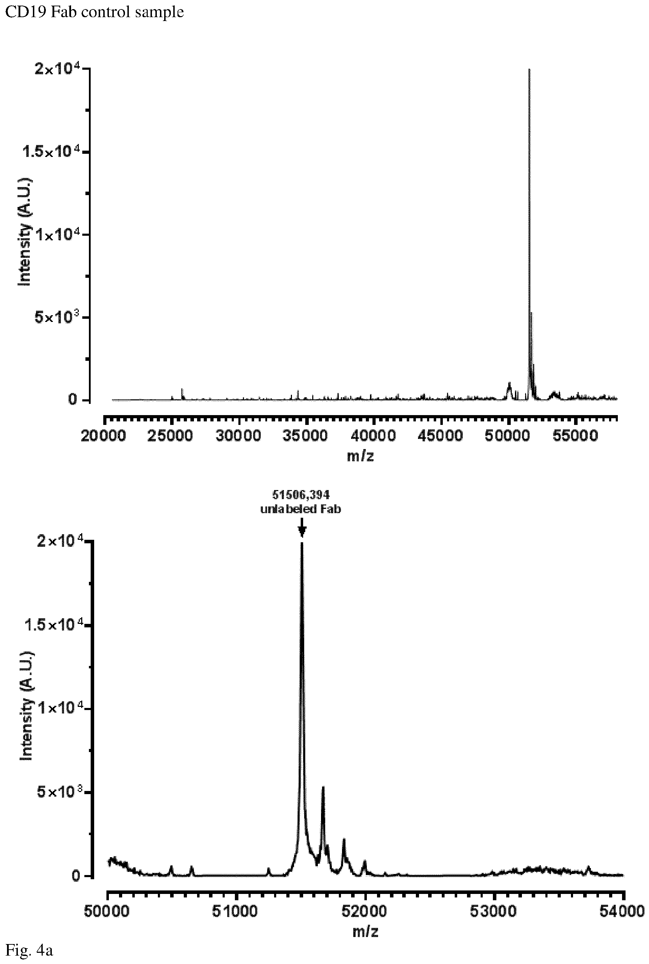

Fig. 4a intact mass determination by microTOF QII of CD19 Fab control sample with MW of 51506,394 Da, showing the value for CD19 Fab with no crosslinker molecule. Fig 4b: intact mass determination data by microTOF QII of CD19 Fab-Biotin after conjugation of crosslinker to Fab via disulphide bond with MW of 52337,281 Da showing the addition of only one crosslinker per Fab molecule and thus obtaining a monofunctional (e.g. with Biotin) Fab.

### Example #2 Biotinylation strategies (stochastic vs site-specific).

CD19 Fab was biotinylated by targeting its lysines with NHS-LCLC-Biotin. The result was compared to biotinylation by targeting the disulphide bond on the C-terminus of the CD19 Fab with the crosslinker which would introduce only one Biotin. For Lysine labelling, CD19 Fab was dissolved in carbonate buffer at pH 8,5. The NHS-LCLC-Biotin reagent was dissolved in DMSO and added in 15 fold molar excess and allow to react for 2 hours, after which the samples was buffer exchanged to PBS. The labelling of CD19 Fab with ETAC crosslinker is described in example 1 (see above). Samples were analysed by LCMS revealing that lysine labelling produces an heterogenous Fab product with species that vary in their degree of labelling (with 2 to 11 biotins per Fab unit) as well as their relative concentration, giving a gaussian distribution which is in correspondence with a stochastic labelling. However, CD19 Fab labelling via the C-terminal disulfide bond deliver only one specie which has only one modification (e.g. Biotin) per Fab, showing how homogenous the product is.

Fig 5a: mass spectroscopy intact mass determination data by microTOF QII of CD19 Fab conjugated with NHS-Biotin, resulting in a stochastic conjugation of multiple biotin units per Fab (from 2 to 11).

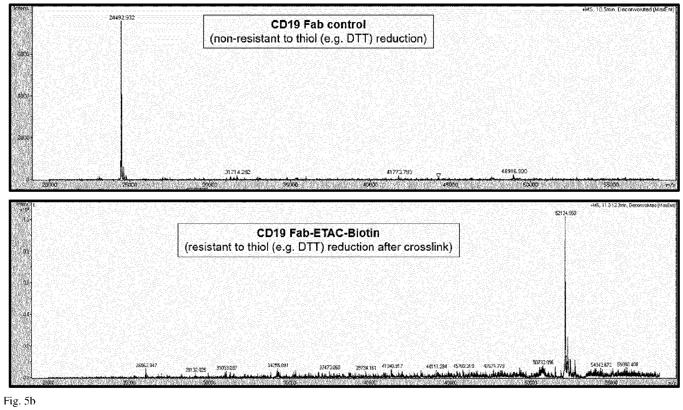

Fig. 5b: intact mass determination data by microTOF QII of CD19 Fab in the presence of thiol reduction environment (e.g. DTT) for: (Top) control, showing no resistance to thiol reduction, and (Bottom) crosslinked with ETAC linker,showing resistance to thiol reduction

### Example #3 showing introduction of different affinity units to Fab

The table below summarizes a list of Fabs of different specificities which have been labelled with different disulphide crosslinkers introducing a variety of affinity units and tags. All samples were analysed by LC-MS and compared to the unmodified control to determine the mass difference and hence the number of affinity units per Fab, which in all cases is always only one. This exemplifies how different affinity units can be used to tag different Fabs.

| anti-human Fab, specificity | Crosslinker mass, dalton | Tag Affinity unit | Mass of unmodified Fab, dalton | Mass of crosslinked Fab, dalton | delta mass, dalton |
|---|---|---|---|---|---|
| CD20 | 832 | DBCO | 47178,148 | 48008,834 | 830,686 |
| CD20 | 511,74 | Azide | 47178,148 | 47691,015 | 512,867 |
| CD20 | 1812,943 | Peptide | 48008,834 | 49822,971 | 1814,137 |
| CD33 | 619 | Biotin | 49752,082 | 50370,331 | 618,249 |
| CD19 | 619 | Biotin | 51506,394 | 52124,958 | 618,564 |
| CD19 | 832 | DBCO | 51506,394 | 52337,993 | 831,599 |
| CD19 | 190,19 | Bimane | 51506,394 | 51696,468 | 190,074 |
| CD133 | 619 | Biotin | 49717,376 | 50337,410 | 620,034 |
| CD53 | 619 | Biotin | 50058,133 | 50677,698 | 619,565 |
| CD34 | 619 | Biotin | 51060,003 | 51679,537 | 619,534 |
| CD4 | 619 | Biotin | 50405,093 | 51023,723 | 618,630 |
| CD8 | 619 | Biotin | 49308,708 | 49928,587 | 619,879 |
| CD56 | 619 | Biotin | 50600,276 | 51218,199 | 617,923 |

### Example #4 showing functionality of the Biotin-crosslinker

Adapter molecule having an antigen recognition moiety CD20 Fab (as shown on Fig 2b)targeting to CD20 antigen on CD20 positive Jeko 1 cells and staining with fluorescent anti-Biotin APC.

CD20 positive Jeko-1 cells were seeded in a 96-well plate (50000 cells/well) and incubated for 10min at 4°C with biotin-crosslinker conjugated anti-CD20 FAb (Rtx Fab MS2) at different concentrations (0,01 -100 µg/ml and 0 µg/ml as negative control) in a total volume of 50 µl Buffer A (CliniMACS PBS/EDTA Buffer + 0.5% BSA). After that 50µl of anti- Biotin APC (1:50 in Buffer A, Miltenyi Biotec, Art. No. 130-110-952) was added, and the sample was further incubated at 4°C for additional 10 min. As positive control anti-human CD20 APC conjugate was used to stain a control sample according to the manufacturer's protocol (Miltenyi Biotec, Art. No. 130-111-525). Finally, 100µl of Buffer A was added to each sample and data was acquired at the MACSQuant Analyzer 10 (Miltenyi Biotec 130-096-343). Frequency of CD20+ cells (gated on only aBio APC as negative control) and median fluorescence intensity of positive cells was analysed.

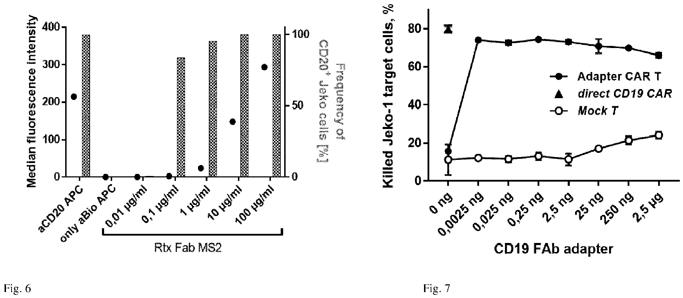

Fig. 6 shows: Titration of adapter molecule using Biotin as Tag, ETAC as linker and CD20 Fab as antigen recognizing domain, on CD20+ Jeko-1 target cells. Different concentrations of FAb (0,01 - 100 µg/ml) was added to 50000 target cells in 50µl and secondary staining was performed with anti-Biotin APC (Miltenyi Biotec). In a control sample direct anti-CD20 staining was performed using an anti-CD20-APC conjugate (Miltenyi Biotec).

Fluorescence and thereby biotinylated FAb bound to target cells was detected at concentration as low as 0,1 µg/ml and median fluorescence intensity increased up to a concentration of 100 µg/ml, indicating that free CD20 binding sites are still available at concentrations of 10 µg/ml, under the conditions used.

### Example #6 showing functionality of killing target cancer cells by combination of adapter CAR T cells and adapter molecule (Fig. 7)

PBMCs were isolated from buffy coat of healthy donors by separation on Ficoll. T cells were selected from PBMCs by MACS technology using the Pan T cell isolation kit (Miltenyi Biotec, 130-096-535). For T cell activation and -expansion the T cells were seeded at 1x106 cells/mL in TexMACS medium containing IL-7 (10 ng/mL) and IL-15 (10 ng/mL) and 1% (v/v) T Cell TransAct, human (Miltenyi Biotec, Art No: 130-111-160) and incubated at 37°C, 5% CO2. Transduction was performed on day 1 after activation. For this, LV supernatant encoding for adapter CARs was added to T cells at a MOI of 5 and cells were carefully resuspended by pipetting up and down. TransAct was removed on day 3 and T cells were further expanded maintaining a cell density of 2x106 cells/mL. On day 6 LNGFR-expressing cells were separated by MACS using anti-LNGFR microbeads on an LS column (Miltenyi Biotec) according to the manufacturer's instructions. Selected, LNGFR+ cells were further expanded up to day 12 and then frozen in aliquots at 1x107 cells/ml in TexMACS + 20% (v/v) FCS + 10% (v/v) DMSO and stored in liquid nitrogen. Aliquots of cells were thawed and cells were washed and recovered in TexMACS containing IL-7 (10 ng/mL) and IL-15 (10 ng/mL) for 48h before the experiment.

Immediately before the experiment GFP transduced Jeko-1 target cells were resuspended in TexMACS without cytokines and 10000 cells were added to each well of a 96-well plate. Then CAR transduced, and untransduced (Mock) T cells were resuspended in TexMACS without cytokines and 50000 cells were added to the respective wells of a 96-well plate. Subsequently, crosslinked and biotinylated CD19 Fab was added to each well at the indicated concentrations and the sample (V = 200 µl) containing Jeko-1 target cells, T cells and Fab, was mixed by carefully pipetting up and down. As a positive control direct anti-CD19 CAR T cells (50000) were co-incubated with 10000 target cells without addition of Fab. All samples were incubated at 37°C, 5% CO2 for 16h. Killing was quantified on a MACSQuant flow cytometer. Propidium iodide was added to the cells immediately before the assay. Killing was evaluated by counting GFP positive and viable (propidium iodide negative) target cells and is expressed as [Killing, %] = [viable GFP+ target cells in untreated sample] / [viable GFP+ target cells in treated sample] x 100 %
Fig. 7 shows a proof-of-concept with the specific killing of target cancer cells (Jeko-1 cells) expressing an antigen recognized by adapter CAR T cells and adapter molecule (i.e. biotin-CD19Fab). The adapter molecule was added in increasing amounts (0-2,5 µg) to 10000 Jeko cells and 50000 CAR T cells (closed circles). Positive control (filled triangle) or negative control Mock (UTD) cells (open circles).

Upon titration of the Fab molecule the Direct CD19 CAR killing after 16h is in the range of 80%. In case of the adapter CAR, maximal killing (75%) is already observed at adapter doses as low as 0,0025 ng in a sample of 200µl (0,0125 ng/ml) and decreased only by approx. 5 % over 6 decades of increasing different adapter concentrations. Furthermore, adapter associated background killing activity with Mock T cells is not detected up to 2,5 ng adapter added in the 200 µl sample (12,5 ng/ml). Overall this indicates that the Fab-based adapter in combination with the adapter CAR T cells enables a high degree of specific lysis (65%) over a broad concentration range covering over 4 decades of adapter concentrations.

### Example #7 unspecific activation of CAR T-cells using biotinylated Ab vs mono-biotinilated Fab

Expression of markers CD25 and CD69 is correlated with activation of T cells, which in turn is related to their cytotoxic effector functions. In order to monitor T cell activation, adapter CAR T cells were incubated for 24h at 37°C and 5% CO2 at increasing concentrations of adapter molecule in presence (closed symbols, E:T = 1:1) or absence (open symbols) of target cells. Two different types of adapter molecule were evaluated: i) Rituximab (anti-CD20 antibody) which was conjugated by NHS-esters and has on average 2-3 Biotin affinity units and the ii) Fab fragment which has 1 Biotin affinity unit/molecule (adapter molecule). Activation of T cells was analysed on a MACSQuant flow cytometer and the fraction of activated cells was defined by the fraction of CD69+ and CD25+ cells. Importantly both adapters efficiently triggered activation of adapter CAR T cells in the presence of target cells in a concentration range of 1x10-10 - 1x10-8 mol/l adapter (50% activation, depending on E:T ratio). However, in absence of target cells only the antibody was able to induce activation of the T cells (maximum activation was observed at a concentration of 1x10-10 mol/l), while monobiotinylated Fab was not able to induce activation of T cells in absence of target cells up to a concentration of 1x10-9 mol/l
Fig. 8 shows monobiotinylated Fabs inducing activation of adapter CAR T cells only in presence of target cells, while adapter molecules based on full length antibodies, and with multiple affinity units, can induce activation of CAR T cells also in absence of target cells. Co-culture (24h) of adapter CAR T and Raji target cells at an effector to target cell ratio of 1. Antibody (Rituximab) or Fab (RituxiFAb) is added at different concentrations (as indicated). The frequency of activated cells (defined by expression of CD69+ and CD25+) is shown.

Activation of CAR T cells in general should be strictly dependent on presence of target cells and cognate adapter molecules, thereby increasing the safety of the approach, activation of T cells in absence of adapter molecules therefore is an undesired property of the adapter which is observed with the whole antibody molecule but not with the monobiotinylated Fab. Use of monobiotinylated Fab as adapter molecule might therefore improve overall safety of the adapter CAR technology.

### Example #8 Monobiotinylated Fab shows increased cytokine secretion from CAR T cells compared to the biotinylated antibody

Use of monobiotinylated Fab molecules can induce efficient release of proinflammatory cytokine (IL-2) from T cells. Different target cells Mel526 (CD20+) and Raji (CD20+) were cocultured in a 96-well plate with adapter CAR T cells at an E:T ratio of 1:1 in 200 µl. Adapter molecules Rituximab- Biotin (n=2-3 Biotin/molecule) and monobiotinylated Rituximab Fab were added at 2x10-11 mol/l, as a negative control non-biotinylated Rituximab was used. After 18h incubation at 37°C and 5% CO2 100µl supernatant was carefully removed from the culture and analysed using the MACSPlex Cytokine 12 Kit, human (Miltenyi Biotec Art. No. 130-099-169) according to the manufacturer's protocol. While in the samples containing only antibody no significant IL-2 release was observed, presence of biotinylated antibody triggered specific cytokine secretion on Mel526 and Raji cells.

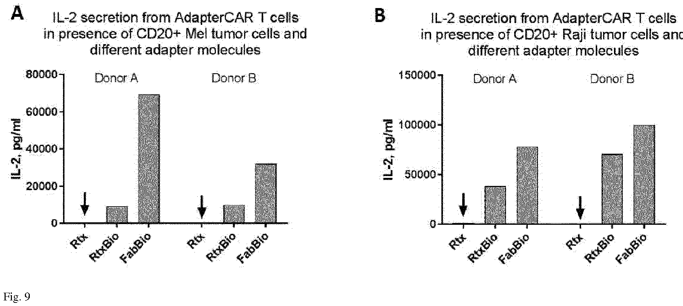

Fig. 9 shows that the use of monobiotinylated Fabs (adapter molecule) can increase target cell specific cytokine release from adapter CAR T cells. Cytokine secretion after 18h of anti-biotin CAR T cells (25.000 cells) in co-culture with A) Mel526 tumor cells expressing CD20 and B) Raji tumor cells, at an effector to target cell ratio of 1:1. Unlabeled Rituximab (Rtx), Rituximab-Biotin (RtxBio) and monobiotinylated Fab (FabBio) were added to the co-culture at 2x10-11 mol/l. On both tumor cell lines, use of the monobiotinylated Fab shows increased cytokine secretion from CAR T cells compare to the biotinylated antibody (Detection limit: 100000 pg/ml).

Notably in presence of the same concentration of biotinylated Fab, cytokine release was increased 3.2-7.4-fold on Mel526 cells and up to 2-fold on Raji cells compared to the biotinylated antibody. Overall this might be attributed to suboptimal orientations (multiple non-productive conformations) on the receptor, which are only possible for the whole antibody molecule having multiple affinity units. Thereby use of a monobiotinylated Fab may improve formation of productive conformations on the adapter CAR T cells in presence of target cells, allowing for an improved immunological synapse formation and cytokine release.

### Example #9 killing assay using Biotinylated Ab with different DOL

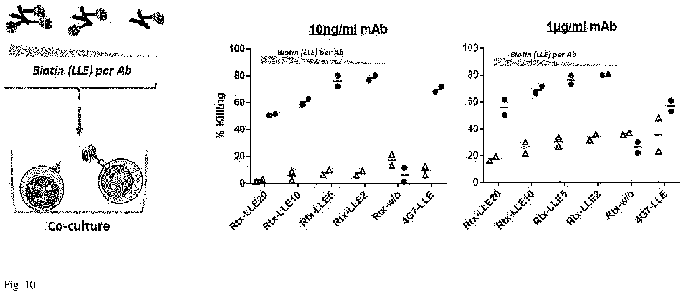

Functionality of adapter molecules having a variable number of affinity units was further addressed in an assay in which Rituximab was conjugated using different amounts of Biotin. The antibody modification by stochastic labeling by succinimidyl-esters at amino groups was done according to protocols well known in the art. For modification, antibodies were rebuffered by passing over an Sephadex G25 column equilibrated in PEB buffer. Collected fractions were assayed for protein content using the Bradford assay. Protein containing fractions were pooled and the total volume determined. Final protein concentration was measured by absorption at 280nm. Subsequently, the corresponding amount of Biotin-LC-LC-NHS (ThermoFisher Scientific, Mw 567,70 g/mol, Cat. No. 21343, CAS-No. 89889-52-1) was dissolved in DMSO. To obtain different degrees of labelling, different amounts of Biotin-LC-LC-NHS was added to reaction mix (3, 6, 12 and 25-fold molar excess). The antibody and DMSO/label mix was incubated at 30°C for 1h and then passed over a Sephadex G25 column. Again fractions were collected, assayed for protein concentration and protein containing fractions were pooled. Final protein concentration was measured by absorption at 280nm. Successful biotinylation was confirmed by LC-MS and by incubation of the antibodies on Jeko-1 cell line expressing the CD20 antibody target and secondary staining with a fluorochrome conjugated anti-biotin antibody, followed by FACS analysis. Rituximab conjugates having a degree of labelling form 2-3 up to 20 biotins/antibody were obtained and subsequently used as adapters used in a killing assay
Fig 10 shows that the low degree of labeling improves adapter (Ab) functionality. CAR T effector cells (red) or Mock (untransduced, blue) T cells were co-cultured with Jeko-1 mantle cell lymphoma (E:T = 5:1, 18h), in presence of Rituximab-Biotin (anti-CD20 Ab) with different degrees of labeling (number of Biotins/Rituximab ranging from 0 (Rtx-LLE-w/o) up to 20 (Rtx-LLE20). As control an anti-CD19 antibody with 6 biotins/molecule was used. Different concentrations of mAb: 1µg/ml and 10ng/ml are shown. Maximal tumor cell lysis after 18h is observed with antibody having a low degree of labeling (average of 2 biotin moieties/Rituximab molecule).

While all biotinylated antibodies were able to mediate killing of target cells, maximum specific target cell lysis was observed with rituximab having a low degree of labelling, with 2-3 biotins per antibody. Notably, no lysines, representing the primary sites of modification for NHS esters can be found in the CDRs of Rituximab. Overall this may highlight importance of using a low degree of labelling on adapter molecules and highlight the importance of monofunctionalized adapters.

### Example #10 determination of tumor burden in live mice which were administrated with CD20Fab-biotin (vs direct CAR, vs Ab, vs Mock, vs Tumor only)

For the in vivo experiments NOD-SCID common γ chain-/- (NSG) mice of female sex were be used, age 7-10 weeks. These are severe combined immunodeficiency (SCID) mice derived on a non-obese diabetic (NOD) background with additional knockout of the common γ-chain (γc-/-). Mice were be obtained from external provider (Jackson labs) and kept in individually ventilated cages (IVC) at 5 animals per cage and group on a standard rodent diet (ssniff, Soest, Germany). Room temperature was constantly kept at 22°C with an air humidity between 50-60%. Light-dark rhythm interval was 12 hours. General health status of all animals will be monitored daily. Raji tumor cells, genetically modified to express firefly luciferase (ffLuc), were transferred by i.v. injection (3x105 cells in 100 µl) and developed systemic leukaemia in the engrafted mice. Tumor progression was regularly monitored (total tumor burden/distribution) by bioluminescence imaging (BLI) in the IVIS (in vivo imaging system). After tumor engraftment for 5 days, adapter CAR T cells and CD20 CAR T cells were be dosed by i.v. injection (1x107 cells per mouse, volume 100 µl) on day 0. Adapter molecules were administered daily by i.p. injection starting on the same day of tumor injection. Administration of adapter molecules was continued for 10 days and tumor progression was continuously monitored.

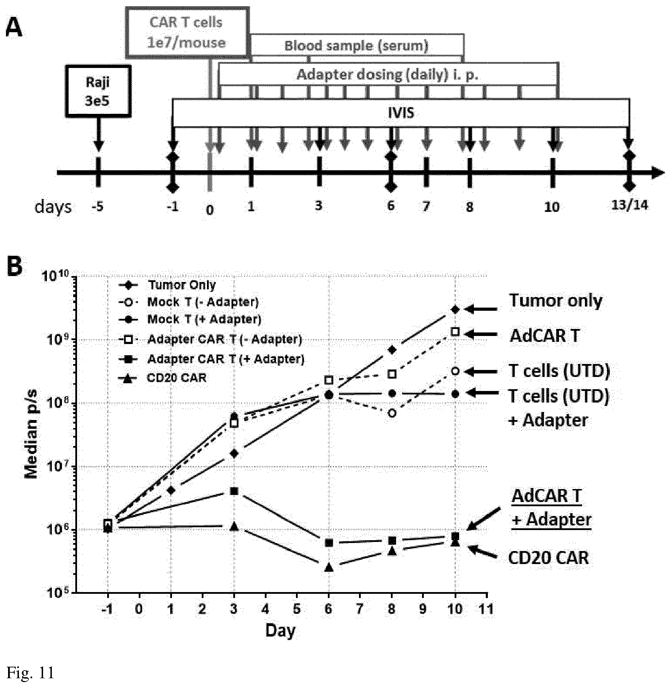

Fig. 11 shows the functionality of anti-affinity unit CAR T cells in presence of crosslinker-modified target cell binding domains (CD20Fab-Biotin) in vivo. NSG mice were engrafted with CD20+ tumor cells (Raji, expressing firefly luciferase) on day-5, CAR T cells were infused on day 0, adapter molecules (50µg/mouse) were administered by i.p. injection on a daily basis. B) Tumor progression (median of groups with n=5 mice) was monitored by bioluminescence imaging (IVIS). Control of tumor growth was only observed in the mice receiving both CAR T cells and adapter molecules and the CD20 direct CAR positive control. While the untreated group which only received tumor cells, BLI was progressing from 1x106 on day 0 to up to 2x109 on day 10, CD20 CAR T cells were able to control tumor outgrowth efficiently with BLI signal of 7x105 on day 10. Adapter CAR T cells in presence of adapter showed a very similar tumor control with median of 8x105 on day 10. In contrast Mock T cells in absence or presence of adapter and adapter CAR T cells in absence of adapter did not control tumor outgrowth, demonstrating that both adapter and adapter CAR T cells need to be present for an efficient tumor control in vivo. Furthermore this experiment highlights the functionality of the monobiotinylated CD20 Fab conjugate in a preclinical setting.

## Claims

1. Use of a cell expressing a chimeric antigen recognition structure (CAR) non-covalently bound to an adapter molecule, wherein:
i) the CAR structure comprises
a) an antigen binding domain specific for the tag of the adapter molecule,
b) a transmembrane domain
c) an intracellular domain
wherein the
ii) the adapter molecule comprises
a) a tag which is specific for the antigen binding domain of the CAR structure
b) a polypeptide comprising an antigen recognizing domain specific for an antigen expressed on the target cell, wherein the polypeptide comprises a heavy chain and a light chain connected by a sulphur atom-linker-sulphur atom bridge and wherein the linker is covalently bound to a single tag
for targeting target cells.

2. Use according to claim 1 **characterized in that the** target cells are tumor cells.

3. Use according to claim 1 or 2 **characterized in that the** target cells originate from a solid tumor.

4. Use according to claim 1 **characterized in that the** target cells originate from tumor microenvironment (TME) of a solid tumor.

5. Use according to claim 1 **characterized in that the** target cells originate from auto-immune disease.

6. Use according to claim 1 **characterized in that the** adapter molecules bind and are specific for soluble antigens.

7. Use according to any of the claims 1 to 6 **characterized in that** the tag is selected from the group consisting of amino acids, peptides, polypeptides, proteins, creatinine, biotin, biocytin, thiamin, fluorochromes, lipids, hormones, other vitamins, carbohydrates.

8. Use according to any of the claims 1 to 7 **characterized in that the** adapter molecule was generated by reducing the disulfide bond between the heavy chain and a light chain of a polypeptide and subsequent reaction with the linker wherein the linker is a molecule consisting of the family of equilibrium transfer alkylation reagents, or the family of dihalogenated maleimides reagents or the family of pyridazedione reagents.

9. Use according to any of the claims 1 to 8 **characterized in that** the cell expressing a chimeric antigen recognition structure (CAR) is a T-Cell, B-cell, NK cell, dendritic cell, tumor infiltrating lymphocyte or macrophage .

10. Use according to any of the claims 1 to 9 **characterized in that** the cell expressing a chimeric antigen recognition structure (CAR) is a T cell; the antigen binding domain specific for the tag is anti-biotin; the tag is biotin; and the linker is derived from the family of equilibrium transfer alkylation reagents, and the polypeptide is an antibody fragment (Fab molecule).

11. Use according to any of the claims 1 to 9 **characterized in that** the cell expressing a chimeric antigen recognition structure (CAR) is a T cell; the antigen binding domain specific for the tag is anti-thiamin; the tag is thiamin; and the linker is derived from the family of equilibrium transfer alkylation reagents, and the polypeptide is an antibody fragment (Fab molecule)
